# EUROPEAN PATENT SPECIFICATION

(11) **EP 3 858 434 B1**
(45) Date of publication and mention of the grant of the patent: **09.12.2026**
(21) Application number: 19851860.7
(22) Date of filing: 06.08.2019
(51) Int. Cl.: A61N 5/01

(54) **RADIOTHERAPY EQUIPMENT**
STRAHLENTHERAPIEGERÄT
ÉQUIPEMENT DE RADIOTHÉRAPIE

(30) Priority: 24.08.2018 CN 201810975999
(43) Date of publication of application: 04.08.2021
(73) Proprietor: Our United Corporation, Xi'an, Shaanxi 710018 (CN)
(72) Inventor: LIU, Haifeng, Xi'an City, Shaanxi 710018 (CN); LI, Daliang, Xi'an City, Shaanxi 710018 (CN)
(74) Representative: Kraus & Lederer PartGmbB
(86) International application number: PCT/CN2019/099390
(87) International publication number: WO 2020/038224

(56) References cited:
- EP-A2- 1 057 499
- WO-A1-2017/020244
- CN-A- 1 275 410
- CN-A- 1 919 373
- CN-A- 101 195 058
- CN-A- 102 698 374
- CN-A- 108 310 684
- CN-A- 109 011 218
- US-B1- 6 512 813

## Description

### TECHNICAL FIELD

The present disclosure relates to the field of medical technologies, and more particularly, to a radiotherapy device.

### BACKGROUND

With the development of medical technologies, radiation treatment is more and more widely used in the treatment of tumors.

In related technologies, a radiotherapy device used for treating heads mainly includes a head gamma knife. Actually, a natural isotope radioactive source, cobalt-60, is utilized to emit gamma rays, and radioactivity of the rays is utilized to kill tumor cells. However, the rays may also damage normal tissues or cells. In the related technologies, the head gamma knife includes 30 or 180 radioactive sources, and a plurality of radioactive sources emit beams from different directions and focus on a common focus, such that the common focus has the largest gamma-ray dose rate, whereas the beams emitted from each radioactive source cause less damage to the normal tissues or cells. In this way, an objective of killing the tumor cells while protecting the normal tissues or cells is achieved, thereby achieving tumor treatment effects.

EP1057499A2 is directed to a whole-body radiotherapy device. Radioactive sources comprise a rotary fixing frame, a source body and a collimator body. The rotating axis is parallel to the longitudinal axis of a treatment couch. A plurality of radioactive sources and their beam channels are distributed in said source body, and the beams from said radioactive sources focus on the common focus point. Collimators are located in the collimator body, and have the same distribution pattern as the radioactive sources. In the radial sectional planes, the radioactive sources and their beam channels are located within a fan shaped area, the included angle of which is less than 90°. In an axial sectional plane, the corresponding angle is preferably less than 60°.

WO2017020244A1 is directed to a focussed radiotherapy apparatus and a radiation therapy device. The radiotherapy apparatus at least comprises: a source body, provided with a plurality of radiation sources; a pre-collimation body, fixedly connected to the source body, the pre-collimation body comprising a plurality of pre-collimation holes, the pre-collimation holes corresponding to the radiation sources in a one-to-one manner; a collimation body, covering the source body and the pre-collimation body and rotating with respect to the source body and the pre-collimation body, the collimation body comprising at least one curved surface, a plurality of groups of collimators, having different specifications, and shielding positions being arranged on the curved surface, each group of collimators and/or shielding positions being arranged in correspondence with the radiation sources, the collimation body rotating to switch different groups of collimators and/or shielding positions to be aligned with the radiation sources so as to obtain different field sizes and/or implement at least partial shielding of the source body; a switch body, arranged on an outer side of the collimation body and moving with respect to the source body, the pre-collimation body and the collimation body so as to enable rays of the radiation sources to be focussed or shielded.

CN101195058A is directed to a radiotherapy apparatus. The radiotherapy apparatus includes a shielding body, a source body provided on the shielding body, an end collimator provided on the shielding body, a shielding body, an intermediate collimator between the end collimator and the source body. The source body, the end collimator and the intermediate collimator can independently rotate relative to the same rotary center line. The end collimator includes multiple groups of through holes with different apertures, and the intermediate collimator includes: at least one group of through holes, at least one group of through holes in high latitude regions, or at least one group of through holes in low latitude regions.

### SUMMARY

The invention relates to a system as defined in the appended claims. Embodiments, examples or aspects in the following disclosure, in particular methods, which do not fall under the scope of the claims are presented for illustration purposes only and do not form part of the invention.

The present disclosure provides a radiotherapy device. The technical solutions are described as follows.

In one aspect, the present disclosure provides a source body. The source body is provided with a plurality of radioactive sources, and an included angle between the plurality of radioactive sources in a longitudinal direction are within a preset included angle range.

In another aspect, the present disclosure provides a radiotherapy device. The radiotherapy device includes a radioactive source apparatus. The radioactive source apparatus includes a source body and a collimator provided by the present disclosure. Beams emitted from the plurality of radioactive sources on the source body intersect at a common focus after being collimated by the collimator.

A radiotherapy device for treating head tumor as recited in claims 1-8 is provided.

### BRIEF DESCRIPTION OF THE DRAWINGS

To describe the technical solutions of the embodiments of the utility model more clearly, the accompanying drawings required for describing the embodiments will be briefly introduced below. Apparently, the accompanying drawings in the following description are merely some embodiments of the present disclosure. To those of ordinary skills in the art, other accompanying drawings may also be derived from these accompanying drawings without creative efforts.
FIG. 1 is a schematic structural diagram of a radiotherapy device in related technologies;
FIG. 2 is a schematic structural diagram of a radioactive source apparatus in related technologies;
FIG. 3 is a schematic structural top view of a source body in related technologies;
FIG. 4 is a schematic diagram of a source body according to an embodiment not covered by the claimed invention;
FIG. 5 is a schematic diagram of another source body according to an embodiment not covered by the claimed invention;
FIG. 6 is a schematic diagram of still another source body according to an embodiment not covered by the claimed invention;
FIG. 7 is a schematic diagram of a source case according to an embodiment not covered by the claimed invention;
FIG. 8 is a schematic structural diagram of a common focus located outside an end surface of the source body according to an embodiment of the present disclosure;
FIG. 9 is a schematic diagram of a radiotherapy device according to an embodiment of the present disclosure;
FIG. 10 is a schematic diagram of another radiotherapy device according to an embodiment of the present disclosure;
FIG. 11 is a schematic diagram of still another radiotherapy device according to an embodiment of the present disclosure;
FIG. 12 is a schematic diagram of still another radiotherapy device according to an embodiment of the present disclosure;
FIG. 13 is a schematic diagram of a control driving method according to an embodiment not covered by the claimed invention;
FIG. 14 is a schematic diagram of a radiation treatment according to an embodiment of the present disclosure;
FIG. 15 is a schematic diagram of another control driving method according to an embodiment not covered by the claimed invention ;
FIG. 16 is a schematic diagram of another radiation treatment according to an embodiment of the present disclosure;
FIG. 17 is a schematic diagram of another control driving method according to an embodiment not covered by the claimed invention ; and
FIG. 18 is a schematic diagram of still another control driving method according to an embodiment not covered by the claimed invention .

### DETAILED DESCRIPTION OF THE EMBODIMENTS

Detailed description of implementations of the present disclosure will further be made with reference to drawings in order to make the above objects, technical solutions and advantages of the present disclosure more apparent.

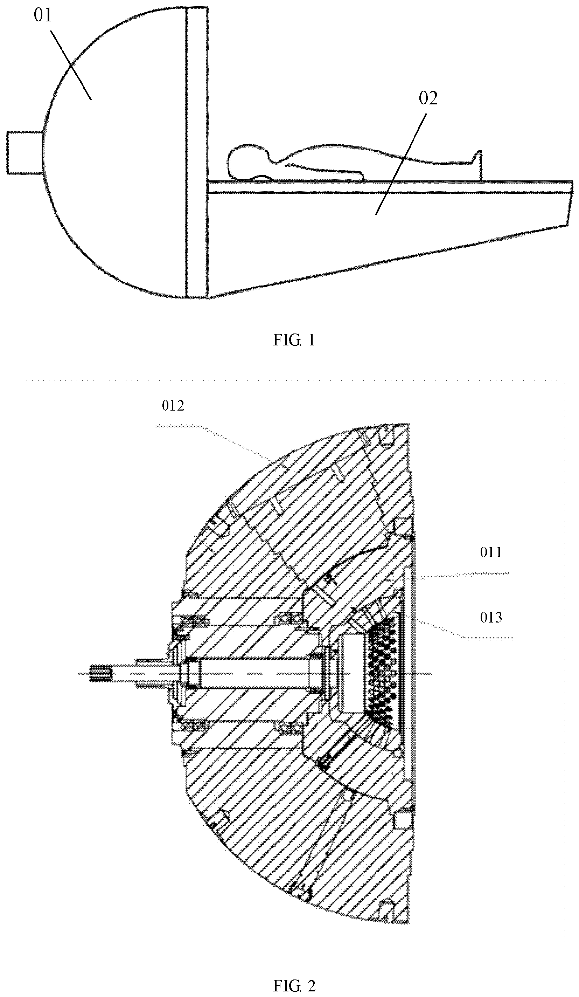

In related technologies, a radiotherapy device used for treating head tumors has a structure for radiation treatment of the head tumors as shown in FIG. 1 and FIG. 2. The radiotherapy device may include a radioactive source apparatus 01 and a treatment couch 02. The radioactive source apparatus 01 may include a source body 011, a shield body 012, and a collimator 013. A plurality of radioactive source (not marked in FIG. 2) may be mounted in the source body 011. Beams emitted from the plurality of radioactive sources intersect at a common focus after passing through a collimation hole (not marked in FIG. 2) on the collimator 02, and the common focus is located in a cavity of the radioactive source apparatus 01. The treatment couch 02 is used to carry a patient and to move the patient into a treatment cabin of the radioactive source apparatus 01, such that a nidus of the patient is located at the common focus for radiation treatment.

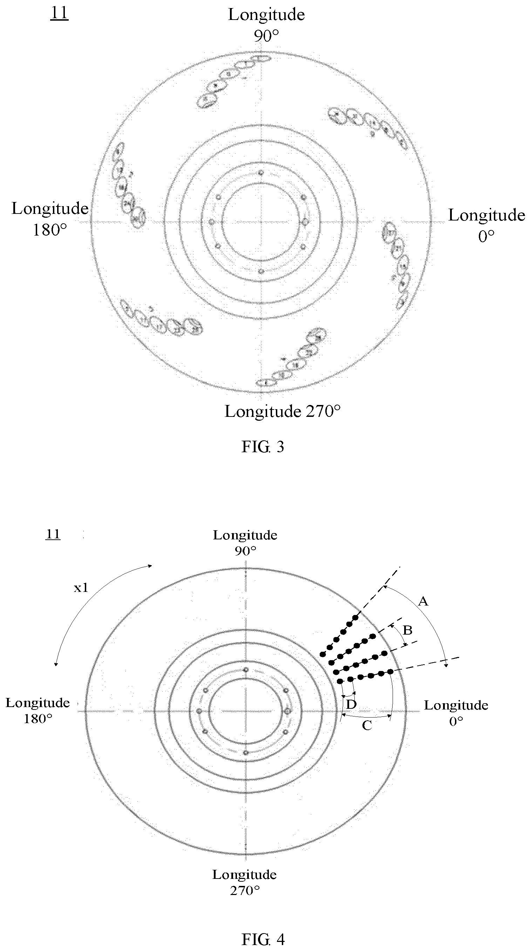

The source body 011 of the radiotherapy device in the related technologies is bowl-shaped as shown in FIG. 3. The radioactive sources in the source body 011 may be divided into six groups, wherein each group includes five radioactive sources, i.e., a total of 30 radioactive sources. The collimator 013 is provided with a plurality of collimation channels, and the beams emitted by the radioactive sources intersect at a common focus after passing through the collimation channels. The collimator 013 includes six collimation channel groups. The six collimation channel groups correspond to the positions of the six groups of radioactive sources. Each collimation channel group includes four subgroups. Collimation holes of one subgroup are filled with solid tungsten rods to implement off source shielding. The other subgroups include five collimation holes, and the collimation holes in different subgroups have different sizes.

During treatment, the source body 011 and the collimator 013 may be driven to rotate with respect to each other to switch the collimation holes of different sizes and to implement on/off source by shielding the radioactive sources by the collimator. However, the six groups of collimation holes having different sizes and the on/off source are switched simultaneously, and one of the groups cannot be controlled individually. Therefore, in the course of treatment, radiation of sensitive tissues and organs (such as eyes and other important nerves) is avoided by adjusting a gamma angle, i.e., by adjusting an elevation angle of head.

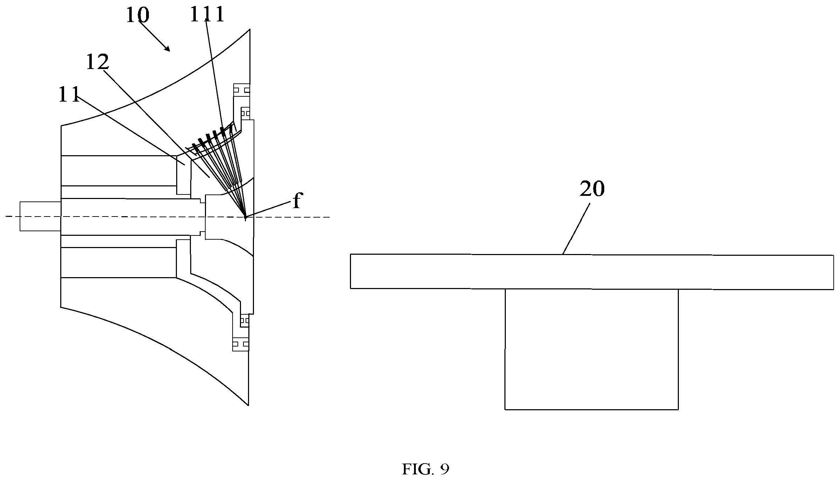

The present disclosure provides a radiotherapy device. Referring to FIG. 9, the radiotherapy device includes a radioactive source apparatus 10. The radioactive source apparatus 10 includes a source body 11 and a collimator 12, the source body 11 is provided with a plurality of radioactive sources 111, an included angle between the plurality of radioactive sources 111 in a longitudinal direction is within a preset included angle range, and beams emitted from the plurality of radioactive sources on the source body 11 intersect at a common focus f after being collimated by the collimator.

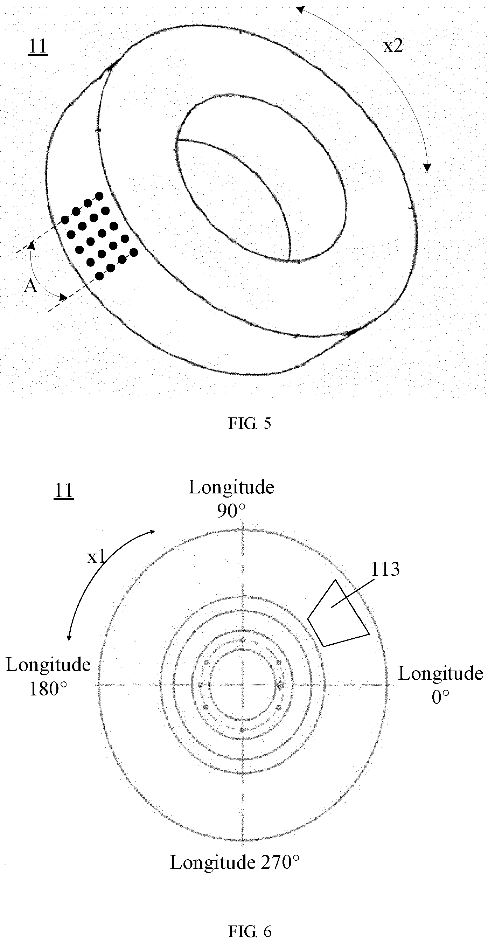

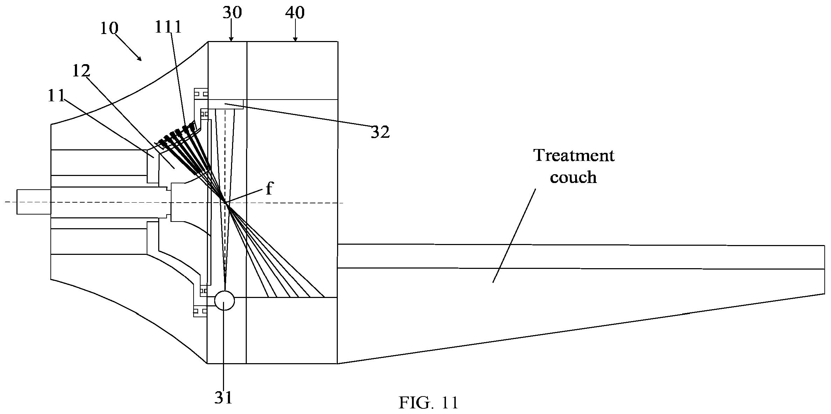

For example, as shown in FIG. 4, the source body 11 may be bowl-shaped, and the longitudinal direction of the source body 11 is as shown by Arrow x1 in FIG. 4, which is a direction of longitude 0°-360°. The source body 11 may also be tube-shaped as shown in FIG. 5 (the radiotherapy device is as shown in FIG. 11), and the longitudinal direction of the source body 11 is the direction as shown by Arrow x2 in FIG. 5. Two ends of a round table in FIG. 5 have equal size, or of course, may have different sizes. The present disclosure does not limit the specific shape of the source body, and the longitudinal direction in the present disclosure is described only taking FIG. 4 and FIG. 5 as examples.

In the present disclosure, the included angle between the radioactive sources in the longitudinal direction is within a preset included angle range, and the maximum included angle between the plurality of radioactive sources in the longitudinal direction is within the preset included angle range. Moreover, in the present disclosure, as shown in FIG. 4, the included angle between the radioactive sources in the longitudinal direction is an included angle formed by using centers of the radioactive sources as a reference. It is to be particularly noted here that if the radioactive sources include one row and the centers of the plurality of radioactive sources in the same row are located on the same longitude line, it is believed that the included angle between the plurality of radioactive sources in the longitudinal direction is zero degree. In the present disclosure, the preset included angle range is greater than or equal to zero degree.

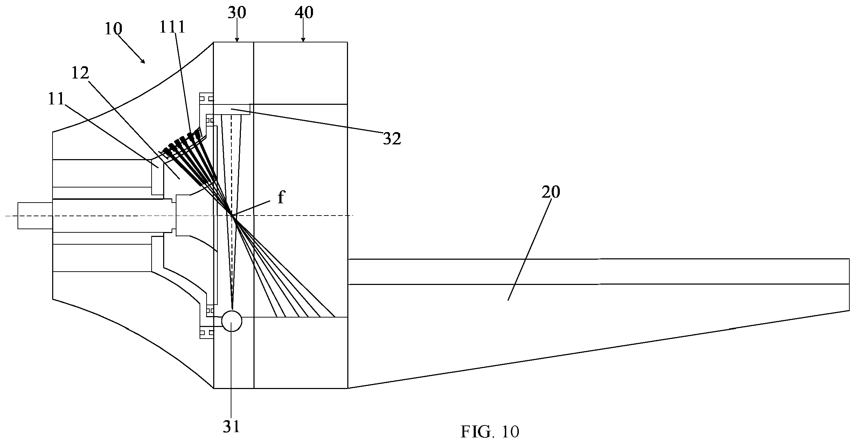

For example, the radiotherapy device provided by the present disclosure may be as shown in FIG. 9 and FIG. 10, the source body 11 is as shown in FIG. 4, and the included angle between a plurality of radioactive sources 111 in the longitudinal direction is denoted as A. For example, the preset included angle range A may be between 5° and 60°, i.e., 5° ≤A≤60°, and the preset included angle range A may be any included angle in the range of from 5° to 60°. For example, the preset included angle range A may be 5°, 8°, 10°, 12°, 18°, 20°, 25°, 30°, 40°, 45°, 50°, or 60°.

A plurality of radioactive sources is arranged on the source body. The number and arrangement of the radioactive sources are not limited in the present disclosure. The number of the radioactive sources may range from 20 to 180, for example, 30 or 180. 24 radioactive sources as shown in FIG. 4 are taken as an example for illustration. The radiotherapy device also includes a collimator. The collimation holes on the collimator correspond to the radioactive sources in number and arrangement, such that the beams emitted from the radioactive sources intersect at a common focus after passing through the collimation holes.

In the radiotherapy device provided by the present disclosure, a plurality of radioactive sources on a source body are distributed within a preset included angle range in a longitudinal direction, and the radioactive sources may be driven by the source body to rotate along a central axis of the radiotherapy device, such that the radioactive sources can be turned off when passing through sensitive tissues or organs, and that the radioactive sources can be turned on when passing through normal tissues and organs. In this way, during the treatment of head tumors, the sensitive tissues and organs such as eyes can be protected from extra damage.

In the radiotherapy device provided by the present disclosure, the radioactive source apparatus 10 further includes a source body driving apparatus configured to drive the source body 11 to rotate about a central axis thereof. The driving apparatus may be a motor, and the radioactive source apparatus 10 may also monitor the driving of the motor to obtain the relative position of the source body 11 in real time so as to determine whether to turn on or off the radioactive sources. The present disclosure does not specifically limit the source body driving apparatus and the position monitoring, and reference may be made to driving technologies in the related technologies, and specific details are omitted herein.

During the treatment, a tumor of a patient may be accurately located at a common focus, such that tumor cells may be killed by radioactive rays. However, if the patient is moved during the treatment, the radioactive rays may deflect, which not only is disadvantageous to the treatment but also is harmful to health of the patient. The common focus of the radiotherapy device in the related technologies is located in a cavity of the radioactive source apparatus, and thus it is impossible to monitor whether the patient's head moves during the treatment. In the radiotherapy device provided by the present disclosure, the common focus is located outside an end surface of the radioactive source apparatus. For example, as shown in FIG. 10 and FIG. 11, the common focus f is located outside the end surface of the radioactive source apparatus, which is advantageous to observe and monitor whether the patient is moved during the treatment.

In the radiotherapy device provided by the present disclosure, the radiotherapy device further includes an imaging apparatus 30. The imaging apparatus 30 is arranged on a side of the radioactive source apparatus 10, and the common focus f is located within an imaging region of the imaging apparatus 30. That is, a tumor of a patient within the imaging region is imaged by the imaging apparatus 30, so to determine whether the patient is moved on the basis of the image. Displacement monitoring based on images has higher accuracy.

For example, the imaging apparatus 30 in the present disclosure may be any combination of one or more of an X-ray imaging apparatus, a CT (Computed Tomography) imaging apparatus, an MR (Magnetic Resonance) imaging apparatus, a DSA (Digital Subtraction Angiography) imaging apparatus, an ultrasound imaging apparatus, or a PET (Positron Emission Computed Tomography) imaging apparatus. For example, the imaging apparatus 30 is the X-ray imaging apparatus. As an example, as shown in FIG. 10, the imaging apparatus 30 may include an X-ray tube 31 and a flat panel detector 32. Alternatively, the imaging apparatus 30 may include two X-ray tubes 31 and two flat panel detectors 32, and beams emitted from the two X-ray tubes 31 intersect. Of course, the imaging apparatus 30 may also be a combination of any two or more different imaging apparatuses. For example, the imaging apparatus may be a combination of the X-ray imaging apparatus and a DSA imaging apparatus. The present disclosure does not limit a specific setup mode of the imaging apparatus, and the above example merely serves as an exemplary description.

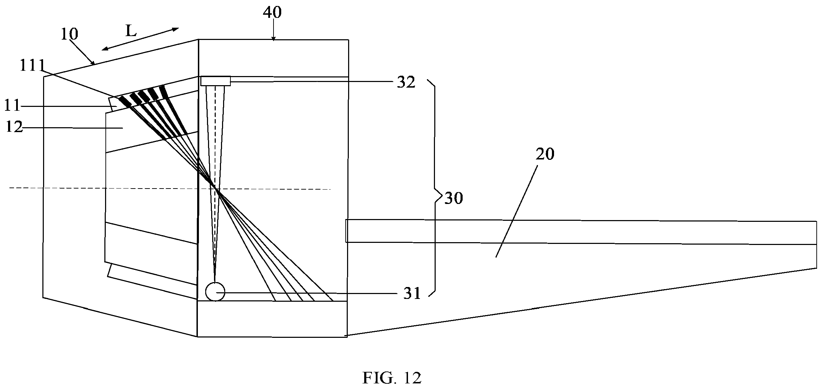

In the radiotherapy device as shown in FIG. 11, the source body 11 of the radiotherapy device is tube-shaped, and two ends of the tube-shaped source body 11 have equal diameter. In the radiotherapy device as shown in FIG. 11, the source body 11 and/or the collimator 12 may also move in a direction as shown by the central axis of the radioactive source apparatus 10 to implement on/off source. In the radiotherapy device as shown in FIG. 12, the source body 11 of the radiotherapy device is tube-shaped, and two ends of the tube-shaped source body 11 have different diameters. In the radiotherapy device as shown in FIG. 12, the source body 11 and/or the collimator 12 may also move in the direction L to implement on/off source. Of course, on/off source may be implemented through rotation, which is not limited in the present disclosure.

As shown in FIG. 10, the imaging apparatus 30 may be a bulb tube 31 and a flat panel detector 32 separately provided with a fixing device to fix the imaging apparatus 30, or the imaging apparatus 30 may also be separately provided with a driving apparatus to drive the bulb tube 31 and the flat panel detector 32 to rotate. Alternatively, as shown in FIG. 11, the imaging apparatus 30 may be fixedly arranged in a shielding apparatus 40, the specific position and structure of the shielding apparatus are not limited in the present disclosure, and the above example merely serves as an exemplary description.

In addition, the radiotherapy device further includes a treatment couch 20 for carrying a patient. As shown in FIG. 9-FIG. 12, the present disclosure does not limit the specific structure or movement form of the treatment couch 20, which may be a three-dimensional couch as shown in FIG. 10-FIG. 12, or may be a six-dimensional couch as shown in FIG. 10. The treatment couch 20 may be selectively configured according to treatment needs, and related details are omitted herein.

In the radiotherapy device provided by the present disclosure, there is further provided with an anti-sinking component between the collimator and the source body. Referring to FIG. 9 to FIG. 10, the anti-sinking component is a bearing.

In the radiotherapy device provided by the present disclosure, the radioactive source apparatus further includes a shielding apparatus 40. The shielding apparatus 40 is located on a side of the radioactive source apparatus 10. Beams emitted from the radioactive source 111 are shielded by the shielding apparatus 40 after passing through the common focus f. For example, as shown in FIG. 10-FIG. 12, the shielding apparatus 40 is located at a side of the common focus f of the radioactive source apparatus 10, and the beams emitted from the radioactive source 111 are shielded by the shielding apparatus 40 after passing through the common focus f, so as to avoid excessive radiation in a treatment room. For example, if the shielding apparatus 40 is shaped like an annular body, all the rays from the radioactive source 111 rotating about the central axis are received by the shielding apparatus 40. Alternatively, the shielding apparatus 40 is a shielding block that can rotate along the central axis of the radioactive source apparatus 10 to follow the radioactive source 111 to rotate so as to receive the rays having passed through the common focus f. It is to be noted that when the treatment couch 20 carries the patient to move, the shielding apparatus 40 is provided with a channel to facilitate the movement of the treatment couch 40.

The source body in the present disclosure is specifically introduced and described below.

The present disclosure provides a source body, wherein the source body 11 is provided with a plurality of radioactive sources 111, and an angle between the plurality of radioactive sources 111 in a longitudinal direction is within a preset angle range. For example, as shown in FIG. 4, the angle between the plurality of radioactive sources in the longitudinal direction is within a preset angle range A. For example, the preset angle range A may be between 5° and 60°, i.e., 5° ≤A≤60°, and the preset angle range A may be any angle in the range of from 5° to 60°. For example, the preset angle range A may be 5°, 8°, 10°, 12°, 18°, 20°, 25°, 30°, 40°, 45°, 50°, or 60°. The number and arrangement of the radioactive sources are not limited in the present disclosure. The number of the radioactive sources generally may range from 20 to 180, for example, 30 or 180. 24 radioactive sources as shown in FIG. 4 are taken as an example for illustration.

For example, in the source body provided by the present disclosure, in the longitudinal direction, the plurality of radioactive sources are classified into a plurality of groups, and the included angle between two adjacent groups of radioactive sources ranges from 2° to 15°. For example, among the plurality of groups of radioactive sources, the included angle between any two adjacent groups of radioactive sources are the same, or the included angles between two adjacent different groups of radioactive sources are different, which is not limited in the present disclosure, and the above example as shown in FIG. 4 merely serves as an exemplary description. As shown in FIG. 4, the plurality of radioactive sources are divided into four rows, taking an example where the included angle between adjacent rows of collimation holes is denoted as B (in FIG. 4, two rows are taken as an example), the included angle B may range from 2° to 15°, i.e., 2° ≤B≤15°, and the preset included angle B may be any included angle in the range of from 2° to 15°. For example, the preset included angle range B may be 2°, 2.5°, 3°, 5°, 6°, 8°, 10°, 12°, or 15°.

In the source body provided by the present disclosure, the included angle between the plurality of radioactive sources in a latitudinal direction ranges from 20° to 60°. For example, as shown in FIG. 4, a plurality of radioactive sources are provided within a preset range C of the source body 11 in the longitudinal direction. For example, the preset included angle range C may be between 20° to 60°, i.e., 20° ≤C≤60°, and the preset included angle range C may be any included angle in the range of from 20° to 60°. For example, the preset included angle range C may be 20°, 25°, 30°, 38°, 40°, 45°, 50°, 53°, or 60°.

For example, in the source body provided by the present disclosure, in the latitudinal direction, the included angle between any two adjacent radioactive sources ranges from 1° to 10°. For example, among the plurality of groups of radioactive sources, the included angle between any two adjacent groups of radioactive sources are the same, or the included angles between any two adjacent groups of radioactive sources are different, which is not limited in the present disclosure, and the above example as shown in FIG. 4 merely serves as an exemplary description. For example, as shown in FIG. 4, taking two radioactive sources as an example, an included angle between the two radioactive sources in the latitudinal direction is denoted as D, the included angle D may range from 1° to 10°, i.e., 1° ≤D≤10°, and the preset included angle D may be any included angle in the range of from 1° to 10°. For example, the preset included angle D may be 1°, 2°, 3°, 5°, 6°, 8°, 9°, or 10°.

For the source body 11 as shown in FIG. 4, an example is taken where the radioactive sources are classified into a plurality of rows in the longitudinal direction and the radioactive sources in the same row have the same longitude, and the radioactive sources are classified into a plurality of rows in the latitudinal direction and the radioactive sources in the same row have the same latitude. Further, to implement non-coplanar radiation and to better protect normal tissues, in the source body provided by the present disclosure, the plurality of radioactive sources has different positions in the latitudinal direction. That is, each radioactive source has different latitudes.

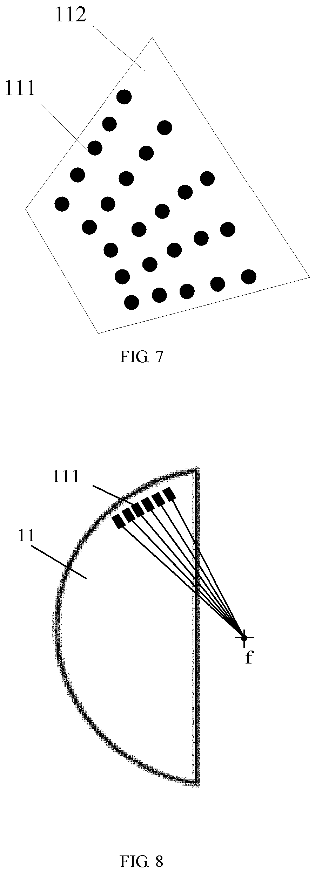

The source body provided by the present disclosure is provided with a plurality of radioactive source holes, and the radioactive sources are fixedly mounted in the radioactive source holes. Alternatively, the source body is provided with a source case position matching a shape of a source case, the source case may be fixedly mounted on the source case position, and the source case is provided with a plurality of radioactive sources. For example, as shown in FIG. 6 and FIG. 7, a plurality of radioactive sources 111 are arranged on the source case 112, and a source case position 113 is arranged on the source body 11. The source case position 113 is internally provided with the source case 112, wherein the source case position 113 may be a through hole or a blind hole, and a plurality of collimation holes are arranged on the source body 11, such that beams from the radioactive source 111 may be emitted through the collimation holes. The present disclosure does not limit shapes or structures of the source case and the source case position, and the above examples as shown in FIG. 6 to FIG. 7 merely serve as an exemplary description.

The source body is further provided with a source case connection portion configured to fix the source case located at the source case position. Similarly, the source case is also provided with a connection portion configured to connect the source case. For example, the source body and the source case may be connected by screws or by snaps. The connection and fixation between the source case and the source case position are not limited in the present disclosure, and the above examples merely serve as an exemplary description.

In the source body provided by the present disclosure, the source case is further provided with a connection portion configured to replace the source case. For example, the connection portion of the source case may be a screw hole, which may be in threaded connection with a source guide rod. Alternatively, the connection portion of the source case may be connected by magnet attraction to the source guide rod. The connection between the source case and the source guide rod and the replacement of the source case are not limited in the present disclosure, and the above example merely serves as an exemplary description.

In the source body provided by the present disclosure, the source case and the source body are formed of different materials. For example, the source case may be formed of tungsten alloy, and the source body may be formed of cast iron.

The source body 11 provided by the present disclosure is bowl-shaped, or generally is also referred to as pan-shaped, helmet-shaped, or hemisphere-shaped, as shown in FIG. 4. Alternatively, the source body 11 may be tube-shaped, as shown in FIG. 5, and diameters of two ends of the tube-shaped source body 11 may be equal or unequal. In FIG. 5, an example is taken where the diameters of the two ends are equal. Alternatively, the source body also may be sheet-shaped. The present disclosure does not specifically limit the specific structure and shape of the source body, and the above example merely serves as an exemplary description.

In the source body provided by the present disclosure, beams emitted from a plurality of radioactive sources intersect at a common focus after being collimated, and the common focus is located on the central axis of the source body, so as to facilitate accurate positioning of the radiotherapy device. The source body may be fixed, or the source body may be driven by a rotary apparatus to rotate 360° circumference or reciprocally about the central axis.

In the source body provided by the present disclosure, the common focus is located outside an end surface of the source body. For example, as shown in FIG. 8, the common focus f is located outside the end surface of the source body 11. The source body 11 is arranged in the entire radiotherapy device, as shown in FIG. 10-FIG. 12, the common focus f is located outside the end surface of the radioactive source apparatus, to facilitate monitoring of a patient or tumor movement.

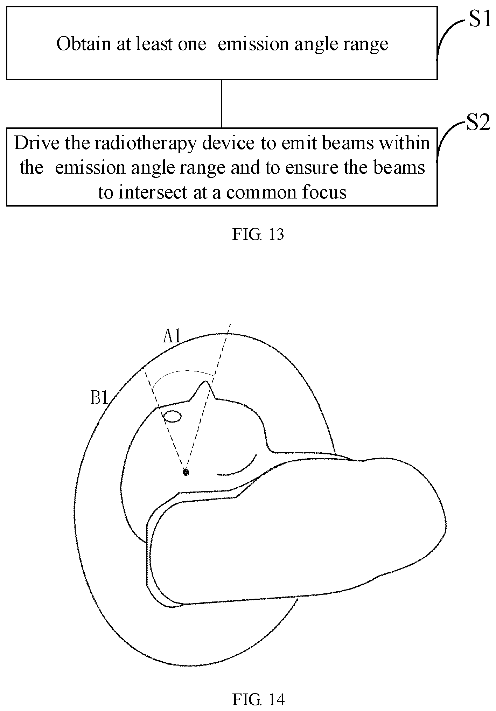

An embodiment not covered by the claimed invention provides a control driving method for a radiotherapy device. The radiotherapy device is the radiotherapy device provided by the present disclosure. For example, the radiotherapy device may be the radiotherapy device as shown in FIG. 9 to FIG. 11. As shown in FIG. 13, this control driving method includes:
Step S1: obtaining at least one beam emission angle range; and
Step S2: driving the radiotherapy device to emit beams within the beam emission angle range and to ensure the beams to intersect at a common focus.

It is to be noted that a preset zero position is generally set for the driving apparatus in the radiotherapy device, and the zero position serves as a reference during the radiation treatment to determine to drive within a drive angle range. In the present disclosure, the beam emission angle range may be an angle range required for the radiotherapy device to emit beams for radiation treatment, wherein the angle range is included in a corresponding treatment plan worked out by a physician according to a tumor image of a patient, and the angle range is an angle range for the driving apparatus to drive. For example, as shown in FIG. 14, in the corresponding treatment plan worked out by the physician according to the tumor image of the patient, the radiotherapy device performs a radiation treatment in a B1 interval, but does not perform the radiation treatment in an A1 interval (an A1 region includes radiation regions of two eyes to prevent rays from causing damage to optic nerves). The beam emission angle range is a drive angle range where the driving apparatus drives the radioactive source to radiate in the B1 interval, and a protection angle range is a drive angle range where the driving apparatus drives the radioactive source not to radiate in the A1 interval. During the radiation treatment, it is only necessary to perform rotatory radiation within the drive angle range of the radiation in the B1 interval, such that sensitive tissues such as the eyes may be prevented from damaging. For example, the drive angle range is a rotation angle of the motor. In the present disclosure, if the radiotherapy device rotates more than 360°, the drive angle range also exceeds 360°. Alternatively, if the radiotherapy device rotates more than 360°, the number of rotations and the drive angle range corresponding to different numbers of rotations are demarcated.

Of course, during the radiation treatment, rotatory radiation may also be performed on the A1 region and the B1 region. In this case, the beam emission angle range is the drive angle range for radiation in the A1 interval and the B1 region. For example, the beam emission angle range may be 360°. At this moment, dosage received by the sensitive tissues such as the optic nerves may be reduced by reducing radiation time to protect the sensitive tissues and organs.

According to a control driving method provided by an embodiment not covered by the claimed invention, a radiotherapy device includes a plurality of radioactive sources, and source points of the plurality of radioactive sources are within a preset angle range in a longitudinal direction. The control driving method includes: obtaining at least one beam emission angle range; and driving the radiotherapy device to emit beams within the beam emission angle range and to ensure the beams to intersect at a common focus, such that sensitive tissues and organs such as eyes can be protected from extra damage during the treatment of head tumors.

As shown in FIG. 5, the control driving method provided by the embodiment not covered by the claimed invention further includes:
Step S3: obtaining at least one protection angle range. The at least one protection angle range is less than 360°.

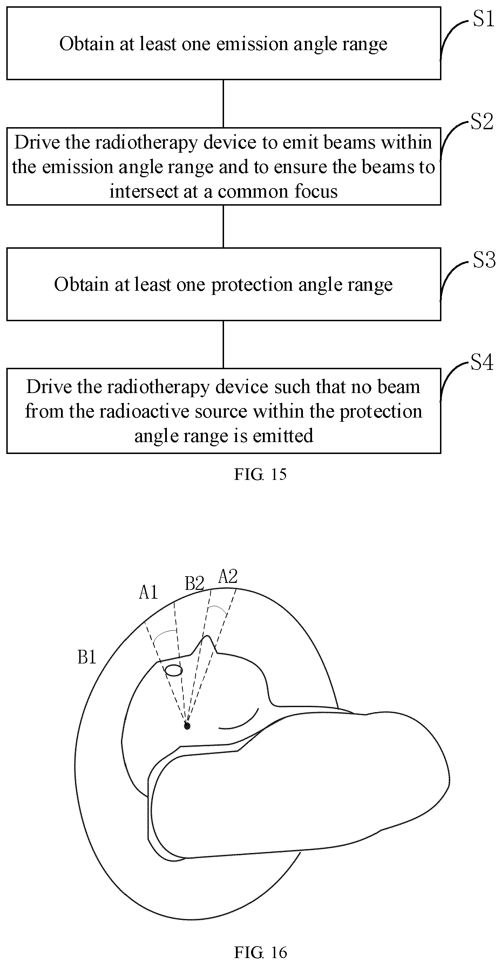

As shown in FIG. 16, the radiotherapy device performs a radiation treatment in a B1 interval and a B2 interval, but does not perform the radiation treatment in an A1 interval and an A2 interval (the A1 interval and the A2 interval correspond to eye regions to prevent rays from causing damage to optic nerves). The beam emission angle range is a drive angle range where the driving apparatus drives the radioactive source to radiate in the B1 interval and the B2 interval, and the protection angle range is a drive angle range where the driving apparatus drives the radioactive source not to radiate in the A1 interval or the A2 interval.

Step S4: driving the radiotherapy device such that no beam from the radioactive source within the protection angle range is emitted.

According to a control driving method provided by an embodiment not covered by the claimed invention, a radiotherapy device includes a plurality of radioactive sources, and source points of the plurality of radioactive sources are within a preset angle range in a longitudinal direction. The control driving method includes: obtaining at least one beam emission angle range and at least one protection angle range; and driving the radiotherapy device to emit beams within the beam emission angle range and to ensure the beams to intersect at a common focus, such that no beam from the radioactive sources within the protection angle range is emitted. In this way, during the treatment of head tumors, the sensitive tissues and organs such as eyes can be protected from extra damage.

For example, at least one of the beam emission angle range is adjacent to one of the protection angle range. As shown in FIG. 16, a radiation treatment is performed in the B1 interval and the B2 interval, but the radiation treatment is neither performed in the A1 interval nor performed in the A2 interval. Because the B1 interval is adjacent to the A1 interval, the beam emission angle range corresponding to the B1 interval is adjacent to the protection angle range corresponding to the A1 interval.

According to the control driving method provided by an embodiment not covered by the claimed invention, the number of the beam emission angle ranges is at least two, and the radiotherapy device has different speeds within the at least two beam emission angle ranges. For example, referring to FIG. 16, when the radiation treatment is performed in the B1 interval and the B2 interval, the beam emission angle range corresponding to the B1 interval and the beam emission angle range corresponding to the B2 interval are obtained. The speed of the radiotherapy device within the beam emission angle range corresponding to the B1 interval is denoted as V1, and the speed of the radiotherapy device within the beam emission angle range corresponding to the B2 interval is denoted as V2, V1≠V2. Radiation time at different positions may be adjusted by adjusting the speed, such that dosage of the focus is adjusted.

For example, as shown in FIG. 14, when a rotatory radiation is performed in the A1 region and the B1 region during the radiation treatment, the beam emission angle range is the drive angle range for radiation performed in the A1 region and the B1 region. The speed of the radiotherapy device within the beam emission angle range corresponding to the B1 interval is denoted as V1, and the speed of the radiotherapy device within the beam emission angle range corresponding to the A1 interval is denoted as V2, V1 <V2. That is, the speed in the A1 interval is greater than the speed in the B1 interval, such that the dosage received by sensitive tissues in the A1 interval is reduced to protect the sensitive tissues and organs.

It is to be noted that the drive angle range in the present disclosure is the rotation angle of the motor, and the drive angle range also exceeds 360°. For example, if the rotation angle of the motor exceeds 360°, the number of rotations and the drive angle range corresponding to different numbers of rotations are demarcated. The radiotherapy device has different speeds within at least two beam emission angle ranges, and has different drive speeds in the same radiation interval corresponding to different numbers of rotations. For example, if planned treatment time for a radiation treatment is 2min, and Imin is required for per rotation of the motor, as shown in FIG. 16, the drive speed for radiation in the B1 region within the beam emission angle range of the first rotation is denoted as V1, and the drive speed for radiation in the B1 region within the beam emission angle range of the second rotation is denoted as V2, V1≠V2.

According to the control driving method provided by an embodiment not covered by the claimed invention, for example, as shown in above figures, two beam emission angle ranges having different speeds are adjacent to each other.

According to the control driving method provided by an embodiment not covered by the claimed invention, the radiotherapy device is driven to reciprocate within the beam emission angle range. For example, if only one beam emission angle range is obtained, the radiotherapy device may reciprocate within the beam emission angle range to increase the dosage received by a tumor. Of course, if a plurality of beam emission angle ranges is obtained, the radiotherapy device may also reciprocate within the beam emission angle range to increase the dosage received by the tumor.

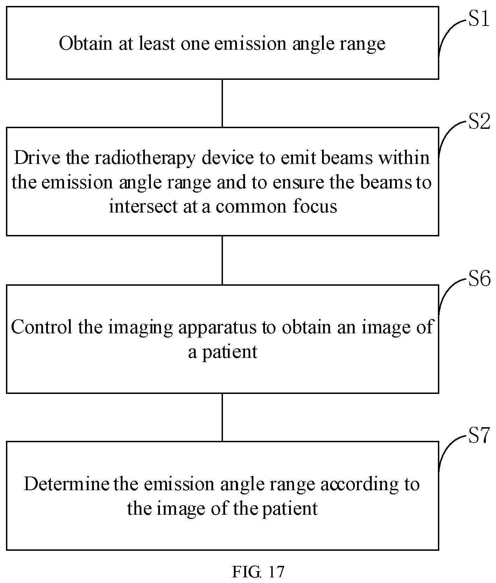

For example, in the radiotherapy device as shown in FIG. 10-FIG. 12, the common focus is located outside an end surface of the radioactive source apparatus. The radiotherapy device further includes an imaging apparatus, and the common focus is located within an imaging region of the imaging apparatus. As shown in FIG. 17, the control driving method also includes:
Step S6: controlling the imaging apparatus to obtain an image of a patient.
Step S7: determining the beam emission angle range according to the image of the patient.

It is to be noted that the beam emission angle range in Step S1 may be a beam emission angle range determined by a physician based on the image of the patient before the radiation treatment, and during the treatment, the beam emission angle range may be determined or adjusted based on the obtained image.

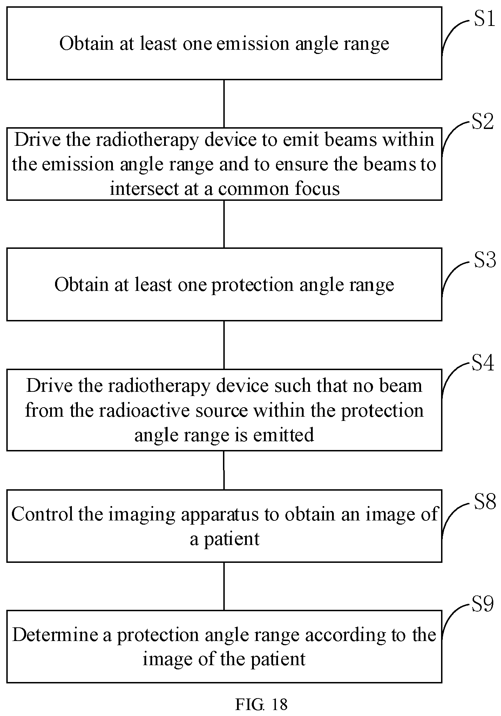

For example, the radiotherapy device as shown in FIG. 10-FIG. 12 further includes an imaging apparatus, and the common focus is located within an imaging region of the imaging apparatus. As shown in FIG. 18, the control driving method also includes:
Step S8: controlling the imaging apparatus to obtain an image of a patient; and
Step S9: determining a protection angle range according to the image of the patient.

Similarly, the protection angle range in Step S3 may be a protection angle range determined by the physician based on the image of the patient before the radiation treatment, and during the treatment, the protection angle range may be determined or adjusted based on the obtained image.

The embodiments described above are merely alternative embodiments of the present disclosure, and are not intended to limit the present disclosur

## Claims

1. A radiotherapy device for treating head tumor, comprising a radioactive source apparatus (10) and an imaging apparatus (30), wherein the radioactive source apparatus (10) comprises a source body (11) and a collimator (12), wherein the source body (11) comprises a plurality of radioactive sources (111), an included angle (A) between the plurality of radioactive sources (111) in a longitude direction is within a preset included angle range, and **characterized in that** beams emitted from the plurality of radioactive sources (111) on the source body (11) intersect at a common focus (f) outside of the radioactive source apparatus (10), the source body (11) has a central axis, the source body (11) rotates around the central axis, the common focus (f) is located on the central axis of the source body (11), the source body (11) has a first end and a second end along the central axis, the imaging apparatus (30) is located on a side of the first end of the source body (11), the common focus (f) is located outside an end surface of the first end of the source body (11) and is located within an imaging region of the imaging apparatus (30).

2. The radiotherapy device according to claim 1, wherein the preset included angle range is 5° to 60°, or the included angle between the plurality of radioactive sources (111) in a latitude direction ranges from 20° to 60°.

3. The radiotherapy device according to claim 1, wherein the source body (11) is provided with a plurality of radioactive source holes, and the radioactive sources (111) are fixedly mounted in the radioactive source holes; or
the source body (11) is provided with a source case position matching a shape of a source case, the source case is fixedly mounted at the source case position, and the source case is provided with the plurality of radioactive sources (111).

4. The radiotherapy device according to claim 1, wherein the source body (11) rotates 360°circumferentiallyor reciprocally around the central axis of the source body (11).

5. The radiotherapy device according to claim 1, wherein the radioactive source apparatus further comprises a source body (11) driving apparatus configured to drive the source body (11) to rotate around the central axis of the source body (11).

6. The radiotherapy device according to claim 1, wherein the radioactive source apparatus (10) further comprises a shielding apparatus (40), the shielding apparatus (40) is located on the side of the first end of the source body (11), and the beams emitted by the radioactive sources (111) are shielded by the shielding apparatus (40) after passing through the common focus (f).

7. The radiotherapy device according to claim 6, wherein the radiotherapy device further comprises a treatment couch (20), and the shielding apparatus (40) is provided with a channel for facilitating movement of the treatment couch (20).

8. The radiotherapy device according to claim 6, wherein the shielding apparatus (40) is a shielding block that can rotate along the central axis of the source body (11) to follow the rotation of the radioactive source (111) so as to receive the beams having passed through the common focus (f).

## Patentansprüche

1. Strahlentherapiegerät zur Behandlung eines Kopftumors, umfassend eine radioaktive Quellenvorrichtung (10) und eine Bildgebungsvorrichtung (30), wobei die radioaktive Quellenvorrichtung (10) einen Quellenkörper (11) und einen Kollimator (12) umfasst, wobei der Quellenkörper (11) mehrere radioaktive Quellen (111) umfasst, wobei ein eingeschlossener Winkel (A) zwischen den mehreren radioaktiven Quellen (111) in einer Längengradrichtung innerhalb eines voreingestellten eingeschlossenen Winkelbereichs liegt, **dadurch gekennzeichnet, dass** sich Strahlen, die von den mehreren radioaktiven Quellen (111) auf dem Quellenkörper (11) emittiert sind, in einem gemeinsamen Brennpunkt (f) außerhalb der radioaktiven Quellenvorrichtung (10) schneiden, wobei der Quellenkörper (11) eine zentrale Achse aufweist, wobei sich der Quellenkörper (11) um die zentrale Achse dreht, wobei sich der gemeinsame Brennpunkt (f) auf der zentralen Achse des Quellenkörpers (11) befindet, wobei der Quellenkörper (11) ein erstes Ende und ein zweites Ende entlang der zentralen Achse aufweist, wobei sich die Bildgebungsvorrichtung (30) auf einer Seite des ersten Endes des Quellenkörpers (11) befindet, wobei sich der gemeinsame Brennpunkt (f) außerhalb einer Endfläche des ersten Endes des Quellenkörpers (11) und innerhalb eines Bildgebungsbereichs der Bildgebungsvorrichtung (30) befindet.

2. Strahlentherapiegerät nach Anspruch 1, wobei der voreingestellte eingeschlossene Winkelbereich 5° bis 60° beträgt oder der eingeschlossene Winkel zwischen den mehreren radioaktiven Quellen (111) in einer Breitengradrichtung im Bereich von 20° bis 60° liegt.

3. Strahlentherapiegerät nach Anspruch 1, wobei der Quellenkörper (11) mit mehreren Löchern für radioaktive Quellen versehen ist und die radioaktiven Quellen (111) fest in den Löchern für radioaktive Quellen montiert sind; oder
wobei der Quellenkörper (11) mit einer an eine Form eines Quellengehäuses angepassten Quellengehäuseposition versehen ist, das Quellengehäuse fest an der Quellengehäuseposition montiert ist und das Quellengehäuse mit den mehreren radioaktiven Quellen (111) versehen ist.

4. Strahlentherapiegerät nach Anspruch 1, wobei sich der Quellenkörper (11) um 360° in Umfangsrichtung oder hin- und hergehend um die zentrale Achse des Quellenkörpers (11) dreht.

5. Strahlentherapiegerät nach Anspruch 1, wobei die radioaktive Quellenvorrichtung ferner eine Antriebsvorrichtung für den Quellenkörper (11) umfasst, die dazu eingerichtet ist, den Quellenkörper (11) so anzutreiben, dass er sich um die zentrale Achse des Quellenkörpers (11) dreht.

6. Strahlentherapiegerät nach Anspruch 1, wobei die radioaktive Quellenvorrichtung (10) ferner eine Abschirmvorrichtung (40) umfasst, wobei sich die Abschirmvorrichtung (40) auf der Seite des ersten Endes des Quellenkörpers (11) befindet, und wobei die von den radioaktiven Quellen (111) emittierten Strahlen nach ihrem Durchgang durch den gemeinsamen Brennpunkt (f) durch die Abschirmvorrichtung (40) abgeschirmt werden.

7. Strahlentherapiegerät nach Anspruch 6, wobei das Strahlentherapiegerät ferner eine Behandlungsliege (20) umfasst, und die Abschirmvorrichtung (40) mit einem Kanal zur Erleichterung der Bewegung der Behandlungsliege (20) versehen ist.

8. Strahlentherapiegerät nach Anspruch 6, wobei die Abschirmvorrichtung (40) ein Abschirmblock ist, der sich entlang der zentralen Achse des Quellenkörpers (11) drehen kann, um der Drehung der radioaktiven Quelle (111) zu folgen und so die Strahlen, die durch den gemeinsamen Brennpunkt (f) hindurchgetreten sind, aufzunehmen.

## Revendications

1. Dispositif de radiothérapie pour le traitement de la tumeur au cerveau, comprenant un appareil à source radioactive (10) et un appareil d'imagerie (30), dans lequel l'appareil à source radioactive (10) comprend un corps de source (11) et un collimateur (12), dans lequel le corps de source (11) comprend une pluralité de sources radioactives (111), un angle inclus (A) entre la pluralité de sources radioactives (111) dans une direction longitudinale se trouve dans une plage prédéfinie d'angle inclus, et **caractérisé en ce que** les faisceaux émis par la pluralité de sources radioactives (111) sur le corps de source (11) se croisent à un foyer commun (f) à l'extérieur de l'appareil à source radioactive (10), le corps de source (11) comporte un axe central, le corps de source (11) tourne autour de l'axe central, le foyer commun (f) est situé sur l'axe central du corps de source (11), le corps de source (11) comporte une première extrémité et une seconde extrémité le long de l'axe central, l'appareil d'imagerie (30) est situé sur un côté de la première extrémité du corps de source (11), le foyer commun (f) est situé à l'extérieur d'une surface d'extrémité de la première extrémité du corps de source (11) et est situé dans une région d'imagerie de l'appareil d'imagerie (30).

2. Dispositif de radiothérapie selon la revendication 1, dans lequel la plage prédéfinie d'angle inclus est de 5° à 60°, ou l'angle inclus entre la pluralité de sources radioactives (111) dans une direction de latitude varie de 20° à 60°.

3. Dispositif de radiothérapie selon la revendication 1, dans lequel le corps de source (11) est pourvu d'une pluralité de trous de source radioactive, et les sources radioactives (111) sont montées de manière fixe dans les trous de source radioactive ; ou
le corps de source (11) est pourvu d'une position de boîtier de source correspondant à une forme d'un boîtier de source, le boîtier de source est monté de manière fixe à la position de boîtier de source, et le boîtier de source est pourvu de la pluralité de sources radioactives (111).

4. Dispositif de radiothérapie selon la revendication 1, dans lequel le corps de source (11) tourne de 360° de manière circonférentielle ou tourne de manière alternative autour de l'axe central du corps de source (11).

5. Dispositif de radiothérapie selon la revendication 1, dans lequel l'appareil à source radioactive comprend en outre un appareil d'entraînement pour le corps de source (11) configuré pour entraîner le corps de source (11) à tourner autour de l'axe central du corps de source (11).

6. Dispositif de radiothérapie selon la revendication 1, dans lequel l'appareil à source radioactive (10) comprend en outre un appareil de blindage (40), l'appareil de blindage (40) est situé sur le côté de la première extrémité du corps de source (11), et les faisceaux émis par les sources radioactives (111) sont blindés par l'appareil de blindage (40) après avoir passé par le foyer commun (f).

7. Dispositif de radiothérapie selon la revendication 6, dans lequel le dispositif de radiothérapie comprend en outre une table de traitement (20), et l'appareil de blindage (40) est pourvu d'un canal pour faciliter le déplacement de la table de traitement (20).

8. Dispositif de radiothérapie selon la revendication 6, dans lequel l'appareil de blindage (40) est un bloc de blindage configuré pour tourner le long de l'axe central du corps de source (11) pour suivre la rotation de la source radioactive (111) afin de recevoir les faisceaux ayant passé par le foyer commun (f).
